# EUROPEAN PATENT APPLICATION

(11) **EP 4 656 727 A1**
(43) Date of publication of application: **03.12.2025**
(21) Application number: 24777967.1
(22) Date of filing: 25.03.2024
(51) Int. Cl.: C12N 15/85, C12N 15/62, C12N 5/10, C12N 15/65

(54) **NUCLEIC ACID, RECOMBINANT VECTOR, TARGETED INTEGRATED CELL, GENE EXPRESSION METHOD, AND USE**

(30) Priority: 28.03.2023 CN 202310347496; 09.11.2023 CN 202311493439
(71) Applicant: Shenzhen Taili Biotechnology Co., Ltd, Shenzhen, Guangdong 518017 (CN)
(72) Inventor: CHEN, Liang, Shenzhen, Guangdong 518017 (CN); YANG, Xiaoli, Shenzhen, Guangdong 518017 (CN); LI, Wenzheng, Shenzhen, Guangdong 518017 (CN); LIN, Longzhi, Shenzhen, Guangdong 518017 (CN); LAN, Wanjun, Shenzhen, Guangdong 518017 (CN); LIANG, Guolong, Shenzhen, Guangdong 518017 (CN)
(74) Representative: Valet Patent Services Limited
(86) International application number: PCT/CN2024/083549
(87) International publication number: WO 2024/199179

(57) **Abstract**

The present invention relates to the field of biotechnology, and in particular to a nucleic acid and a recombinant vector and targeted integrated cell comprising same, and a method for producing a target gene expression product and a use thereof. The nucleic acid comprises a nucleic acid fragment shown in SEQ ID No.1, and the nucleic acid fragment is used for integrating an exogenous nucleic acid fragment. Upon a large number of cell strain screenings in the early stage, it is found that for the nucleic acid fragment as shown in SEQID No.1 or a homologous fragment that maintains at least 90% identity with the nucleic acid fragment, an exogenous nucleotide sequence is integrated into the nucleic acid fragment or the homologous fragment thereof, and the correspondingly obtained targeted integrated cell has the characteristics of high identity, high stability, and high yield.

## Description

### CROSS REFERENCE TO RELATED APPLICATIONS

The present application claims the priority both to the Chinese patent application with the filing No. 2023103474969, entitled "NUCLEIC ACID, RECOMBINANT VECTOR, TARGETED INTEGRATED CELL, GENE EXPRESSION METHOD AND USE" and filed on March 28, 2023 with the Chinese Patent Office, and to the Chinese patent application with the filing No. 2023114934398, entitled "NUCLEIC ACID, RECOMBINANT VECTOR, TARGETED INTEGRATED CELL, GENE EXPRESSION METHOD AND USE" and filed on November 9, 2023 with the Chinese Patent Office, the entire contents of which are incorporated herein by reference in their entirety.

### TECHNICAL FIELD

The present application relates to the field of biotechnology, and particularly to a nucleic acid and a recombinant vector containing the same, a targeted integrated cell, a method for producing a target gene expression product and a use.

### BACKGROUND ART

Host cells (such as Chinese hamster ovary cells) have always been the main standard expression platform for producing recombinant proteins. The traditional method for developing suitable host cells includes randomly inserting the target gene into the genome and then selectively culturing the cells carrying the transgene. However, in the implementation process of the traditional method: in the cell line generation stage of the upstream process, a non-targeted transgenic integration method is used to generate a stably transfected cell pool, and then multiple clones are obtained by monoclonal means, and then multiple clones are subjected to tedious screening to determine the clones with suitable production characteristics; and in subsequent processes, process development and culture medium optimization are required for each strain of cells, entailing substantial repetitive work and high costs, and resulting in waste of resources. In terms of the prepared cell line quality, the expression stability of the cell lines obtained by this method is difficult to predict during the culture process. Moreover, the information of random integration sites is unclear, and the site effect of exogenous target gene integration may also cause significant decrease in the expression level of the target gene.

In general, the traditional random integration method is used for constructing cell lines, which usually takes more than 6 months to obtain a stable cell strain. Random integration has drawbacks such as enormous workload, extremely high cost, unstable copy numbers and sites, and uncertainties regarding the impact of sites on cell physiology, which all may lead to that the produced cell strains lose target genes during the growth and thereby lose production values.

### SUMMARY

One of the purposes of embodiments of the present application includes providing a nucleic acid, where a host cell including the nucleic acid may stably and highly express an exogenous nucleic acid fragment integrated into SEQ ID No. 1.

In the first aspect of the present application, a nucleic acid is provided, where the nucleic acid includes the nucleic acid fragment as shown in SEQ ID No. 1, and the nucleic acid fragment is used to integrate an exogenous nucleic acid fragment.

In some embodiments of the present application, the integration site of the exogenous nucleic acid fragment corresponds to any site within a base interval from positions 11 to 430 of the nucleic acid fragment.

In some embodiments of the present application, the integration site of the exogenous nucleic acid fragment corresponds to any site within the base interval from positions from 21 to 414 of the nucleic acid fragment.

In some embodiments of the present application, the integration site of the exogenous nucleic acid fragment corresponds to any site within the base interval from positions from 38 to 402 of the nucleic acid fragment.

In some embodiments of the present application, the integration site of the exogenous nucleic acid fragment corresponds to any site within the base interval from positions from 53 to 389 of the nucleic acid fragment.

In some embodiments of the present application, the integration site of the exogenous nucleic acid fragment corresponds to any site within the base interval from positions from 73 to 373 of the nucleic acid fragment.

In some embodiments of the present application, the integration site of the exogenous nucleic acid fragment corresponds to any site within the base interval from positions from 91 to 360 of the nucleic acid fragment.

In some embodiments of the present application, the integration site of the exogenous nucleic acid fragment corresponds to any site within the base interval from positions from 108 to 342 of the nucleic acid fragment.

In some embodiments of the present application, the integration site of the exogenous nucleic acid fragment corresponds to any site within the base interval from positions from 126 to 326 of the nucleic acid fragment.

In some embodiments of the present application, the integration site of the exogenous nucleic acid fragment corresponds to any site within the base interval from positions from 143 to 310 of the nucleic acid fragment.

In some embodiments of the present application, the integration site of the exogenous nucleic acid fragment corresponds to any site within the base interval from positions from 160 to 295 of the nucleic acid fragment.

In some embodiments of the present application, the integration site of the exogenous nucleic acid fragment corresponds to any site within the base interval from positions from 178 to 274 of the nucleic acid fragment.

In some embodiments of the present application, the integration site of the exogenous nucleic acid fragment corresponds to any site within the base interval from positions from 194 to 263 of the nucleic acid fragment.

In some embodiments of the present application, the integration site of the exogenous nucleic acid fragment corresponds to any site within the base interval from positions from 209 to 253 of the nucleic acid fragment.

In some embodiments of the present application, the integration site of the exogenous nucleic acid fragment corresponds to any site within the base interval from positions from 221 to 242 of the nucleic acid fragment.

In some embodiments of the present application, the integration site of the exogenous nucleic acid fragment corresponds to any site within the base interval from positions from 231 to 240 of the nucleic acid fragment.

In some embodiments of the present application, the integration site of the exogenous nucleic acid fragment corresponds to the NW_003616785.1:83044 site within the nucleic acid fragment.

In some embodiments of the present application, the integration site of the exogenous nucleic acid fragment corresponds to the NW_003616785.1:83044 site within the nucleic acid fragment in the CHO (Chinese hamster ovary) cell.

In some embodiments of the present application, the exogenous nucleic acid fragment includes a first recombination recognition sequence, a second recombination recognition sequence, a selection marker gene and/or a target gene located between the first recombination recognition sequence and the second recombination recognition sequence, and a promoter that regulates the expression of the selection marker gene and/or the target gene.

In some embodiments of the present application, the exogenous nucleic acid fragment includes a selection marker gene located between the first recombination recognition sequence and the second recombination recognition sequence, and a promoter that regulates the expression of the selection marker gene.

In some embodiments of the present application, the exogenous nucleic acid fragment includes a target gene located between the first recombination recognition sequence and the second recombination recognition sequence, and a promoter that regulates the expression of the target gene.

In some embodiments of the present application, the exogenous nucleic acid fragment includes a selection marker gene and a target gene located between the first recombination recognition sequence and the second recombination recognition sequence, and a promoter that regulates the expression of the selection marker gene and the target gene.

In some embodiments of the present application, the first recombination recognition sequence and the second recombination recognition sequence are generated by a recombinase; optionally, the recombinase is a Bxb1 integrase, a ΦC31 integrase, a Cre recombinase or a FLP recombinase; optionally, the recombinase is the Bxb1 integrase;
or/and
the first recombination recognition sequence and the second recombination recognition sequence are each independently selected from one or more of following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxb1 attP sequence, a Bxb1 attB sequence, an attP sequence, an attB, an attL and an attR sequence; optionally, the first recombination recognition sequence and the second recombination recognition sequence are each independently selected from the attR sequence and the attL sequence; optionally, the first recombination recognition sequence is the attR, and the second recombination recognition sequence is the attL. In some embodiments of the present application, the selection marker gene is one or more selected from a neomycin resistance gene, a thymidine kinase gene, a hygromycin phosphotransferase gene, a dihydrofolate reductase gene, a thymidine kinase gene, a glutamine synthetase gene, an asparagine synthetase gene, a tryptophan synthetase gene, a histamine alcohol dehydrogenase gene, an aminoglycoside phosphotransferase gene, a tryptophan synthetase gene and a fluorescent protein gene; optionally, the selection marker gene is the fluorescent protein gene.

In some embodiments of the present application, the promoter is a CMV promoter, a SV40 promoter, an RSV promoter, a β-globin promoter, a UBC promoter, an EF1a promoter, a ubiquitin promoter, a β-actin promoter, a PGK1 promoter, a Rosa26 promoter, a HSP70 promoter, a GAPDH promoter, an Eif4A1 promoter, an Egr1 promoter, a FerH promoter, a SM22α promoter or an Endothelin-1 promoter.

In some embodiments of the present application, the target gene encodes one or more of an antibody, a recombinant protein, a polypeptide, an enzyme, a hormone, a growth factor and a receptor; optionally, the target gene encodes the antibody; and optionally, the nucleotide sequence of the target gene is as shown in SEQ ID No. 2.

In some embodiments of the present application, the nucleotide sequence of the nucleic acid includes the nucleotide sequence as shown in SEQ ID No. 3.

In some embodiments of the present application, the nucleotide sequence of the nucleic acid includes the nucleotide sequence as shown in SEQ ID No. 7.

In some embodiments of the present application, the nucleotide sequence of the nucleic acid includes the nucleotide sequence as shown in SEQ ID No. 9.

In the second aspect of the present application, a recombinant vector is provided, which includes the nucleic acid according to the first aspect, where the nucleic acid further includes the first recombination recognition sequence and the second recombination recognition sequence, and the exogenous nucleic acid fragment includes a target gene and/or a selection marker gene.

In some embodiments of the present application, the exogenous nucleic acid fragment is a target gene; the first recombination recognition sequence is a 5' homology arm homologous to the sequence fragment present in the nucleic acid fragment as shown in SEQ ID No. 1; the second recombination recognition sequence is a 3' homology arm homologous to the sequence fragment present in the nucleic acid fragment as shown in SEQ ID No. 1; optionally, the 5' homology arm is the attR and the 3' homology arm is the attL.

In some embodiments of the present application, the exogenous nucleic acid fragment is a selection marker gene; the first recombination recognition sequence is a 5' homology arm homologous to the sequence fragment present in the nucleic acid fragment as shown in SEQ ID No. 1; the second recombination recognition sequence is a 3' homology arm homologous to the sequence fragment present in the nucleic acid fragment as shown in SEQ ID No. 1; and optionally, the 5' homology arm is attP, and the 3' homology arm is attP-GA.

In some embodiments of the present application, the recombinant vector is a lentiviral vector, an adenoviral vector, an adeno-associated viral vector, a herpesvirus vector, a poxvirus vector, a baculovirus vector, a papillomavirus vector, a papovavirus vector, an integrating phage vector, a non-viral vector, a transposon and/or a transposase, an integrase substrate or a plasmid.

In the third aspect of the present application, a targeted integrated cell is provided, where the targeted integrated cell includes the nucleic acid according to the first aspect.

In some embodiments of the present application, the targeted integrated cell is a eukaryotic cell;
In some embodiments of the present application, the eukaryotic cell is a mammalian cell;
In some embodiments of the present application, the mammalian cell include Chinese hamster ovary CHO cell and human embryonic kidney HEK293 cell; and optionally, the targeted integrated cell is the Chinese Hamster Ovary CHO cell.

In the fourth aspect of the present application, a preparation method for the targeted integrated cell according to the third aspect is provided. The preparation method includes the following steps:
introducing the nucleic acid according to the first aspect into a cell, or providing a cell including the nucleic acid fragment as shown in SEQ ID No. 1 and integrating the exogenous nucleic acid fragment into the nucleic acid fragment to prepare the targeted integrated cell.

In the fifth aspect of the present application, a method for producing a target gene expression product is provided, where the method includes the following steps: culturing the targeted integrated cell according to the third aspect, and collecting the expression product of the target gene in the exogenous nucleic acid fragment.

In the sixth aspect of the present application, a use of the nucleic acid according to the first aspect in preparing a protein or a polypeptide is provided, where the protein or the polypeptide is expressed by the exogenous nucleic acid fragment.

Compared with the traditional technique, the present application has the following beneficial effects.

In the early stage of the present application, a nucleic acid fragment as shown in SEQ ID No. 1 or a homologous fragment with at least 90% consistency therewith is found after screening a large number of cell strains, the exogenous nucleotide sequence (such as a fusion protein and a monoclonal antibody) is integrated into the nucleic acid fragment or the homologous fragment thereof, and the corresponding targeted integrated cells obtained are characterized by high consistency, stability and high yield. It is precisely because of these advantages that the subsequent culture processes (such as culture conditions and medium formulations) do not require high standards.

Meanwhile, the present application provides a targeted integrated cell, the insertion site of the exogenous nucleotide fragment is determined, so compared with the traditional cell construction method involving random integration, the construction process required for the targeted integrated cell of the present application is simple, and has shortened time consumption, improved efficiency, reduced cost, lowered uncertainty, good repeatability and strong controllability.

### BRIEF DESCRIPTION OF DRAWINGS

In order to more clearly illustrate the technical solutions in the embodiments of the present application and to more fully understand the present application and its beneficial effects, the drawings required to be used in the description of the embodiments will be briefly introduced below. Obviously, the drawings described below are only some examples of the present application, and a person skilled in the art may obtain other drawings based on these drawings without paying creative work.
FIG. 1 is a process flow chart in embodiments of the present application;
FIG. 2 is an integration site selection plasmid map in embodiments of the present application;
FIG. 3 is a production transfection plasmid map in embodiments of the present application;
FIG. 4 is a statistical diagram of the protein expression level of the cell integrated with the target gene 1 in embodiments of the present application;
FIG. 5 is a verification diagram of continuous passage stability of the cell integrated with the target gene 1 in embodiments of the present application;
FIG. 6 is an electrophoresis diagram of the cell integrated with the target gene 1 in embodiments of the present application; and
FIG. 7 is an electrophoresis diagram of the cell integrated with the target genes 2 and 3 in embodiments of the present application.

### DETAILED DESCRIPTION OF EMBODIMENTS

The present application will be further described in detail below in conjunction with the drawings, embodiments and examples. It should be understood that these embodiments and examples are only used to illustrate the present application and are not used to limit the scope of the present application. It should also be understood that the present application may be implemented in many different forms and is not limited to the embodiments and examples describe herein, a person skilled in the art may make various changes or modifications without violating the connotation of the present application, and the equivalent forms obtained also fall within the protection scope of the present application. In addition, in the following description, a large number of specific details are given to provide a more comprehensive understanding of the present application, and it should be understood that the present application may be implemented without one or more of these details.

Unless otherwise defined, all technical and scientific terms used herein have the same meaning as those commonly understood by those skilled in the art to which the present application belongs. The terms used herein in the specification of the present application are only for the purpose of describing embodiments and examples and are not intended to limit the present application.

### Terms

Unless otherwise specified or incompatible herewith, the terms and phrases used herein should have the following meanings.

The selection range of the terms "and/or", "or/and", and "as well as/or" used herein include any one of two or more than two of the related listed items, as well as any and all combinations of the related listed items, where the any and all combinations include combinations of any two related listed items, any more related listed items, or all related listed items. It should be noted that when at least three items are combined and connected by at least two conjunctions selected from "and/or", "or/and", "as well as/or", it should be understood that in the present application, the technical solutions undoubtedly include the technical solutions where all items are connected by "logical AND", and also undoubtedly includes the technical solutions where all items are connected by "logical OR". For example, "A as well as/or B" includes three parallel solutions of A, B, and A+B. For another example, the technical solutions of "A, as well as/or, B, as well as/or, C, as well as/or, D" includes any one of A, B, C, and D (i.e., the technical solutions are all connected by "logical OR"), and also includes any and all combinations of A, B, C, and D, that is, combinations of any two or any three of A, B, C, and D, as well as the combination of all four of A, B, C, and D (i.e., the technical solutions are all connected by "logical AND").

In the present application, "a plurality of", "a variety of", "multiple times", "pluralistic", etc., unless otherwise specified, refer to a quantity greater than 2 or equal to 2. For example, "one or more" means one or two or more.

As used herein, "combination thereof", "any combination thereof", "any combination mode thereof" and the like include all appropriate combination modes of any two or more than two items among the listed items.

As used herein, the term "appropriate" as used in expressions such as "appropriate combination modes", "appropriate modes", and "any appropriate modes" should be based on the ability to implement the technical solutions of the present application, solve the technical problems of the present application, and achieve the expected technical effects of the present application.

As used herein, "preferable", "preferred", "better", and "appropriate" are only used to describe embodiments or examples with better effects. It should be understood that they do not constitute a limitation on the protection scope of the present application.

In the present application, "further", "more further", "particularly" and the like are used for descriptive purposes to indicate differences in content, but should not be construed as limiting the scope of protection of the present application.

In the present application, "optionally", "optional", and "option" mean that something may be present or absent, that means it is any one selected from the two parallel solutions of "present" or "absent". If multiple instances of "option" appear in a technical solution, each "option" is independent from the others, unless otherwise specified and provided that there is no contradiction or mutual restriction between them.

In the present application, the terms "first", "second", "third", and "fourth", etc. in "the first aspect", "the second aspect", "the third aspect", and "the fourth aspect", etc. are used for descriptive purposes only and may not be understood as indicating or implying relative importance or quantity, nor can they be understood as implicitly indicating the importance or quantity of the indicated technical features. Moreover, "first", "second", "third", and "fourth", etc. are merely used for non-exhaustive enumerative description, and it should be understood that they do not constitute a closed limitation on quantity.

In the present application, among the technical features described in an open-ended manner, both the closed-ended technical solutions consisting of the listed features and the open-ended technical solutions containing the listed features are included.

In the present application, with respect to numerical intervals (i.e., numerical ranges), unless otherwise specified, the optional numerical values distributed within the aforesaid numerical intervals are deemed to be continuous, and include both numerical value endpoints of the numerical range (i.e., the minimum value and the maximum value), as well as every numerical value between these two numerical range endpoints. Unless otherwise specified, when a numerical range refers only to integers within the numerical range, it includes the two endpoint integers of the numerical range, and each integer between the two endpoints. As used herein, this is equivalent to directly enumerating each integer. For example, that t is an integer selected from 1 to 10 means that t is any integer selected from the group of integers consisting of 1, 2, 3, 4, 5, 6, 7, 8, 9, and 10. In addition, when multiple ranges are provided to describe features or characteristics, these ranges may be combined. In other words, unless otherwise specified, the ranges disclosed herein should be construed as including any and all subranges subsumed therein.

For the temperature parameters in the present application, unless otherwise specified, both constant temperature treatment and variation present within a certain temperature range are allowed. It should be understood that the constant temperature treatment allows the temperature to fluctuate within the precision range controlled by an instrument, fluctuate within a range of such as ± 5 °C, ± 4 °C, ± 3 °C, ± 2 °C, and ± 1 °C.

In the present application, %(w/w) and wt% both indicate weight percentage, %(v/v) indicates volume percentage, and %(w/v) indicates mass volume percentage.

In the present application, "about" or "approximately" means within an acceptable error range for a particular value as determined by a person ordinarily skilled in the art, which depends in part on how the value is measured or determined, i.e., the limitations of the measurement system. For example, according to practice in the art, "about" may mean within 3 or more standard deviations. Alternatively, "about" may mean a range of up to 20%, preferably up to 10%, more preferably up to 5%, and even more preferably up to 1% of a given value. Alternatively, particularly with respect to biological systems or processes, the term may mean within an order of magnitude of the value, preferably within 5 times, and more preferably within 2 times.

In the present application, the "selection marker gene" may be a gene as follows, where the gene allows, in the presence of a corresponding selective agent, the targeted integrated cell carrying the gene to be specifically selected either for or against the gene. For example, but non-limitedly, the selection marker may allow, in the presence of the gene, the targeted integrated cell transformed with the selection marker gene to be positively selected; non-transformed targeted integrated cell may be unable to grow or survive under the selection conditions. The selection markers may be positive, negative, or bifunctional. Positive selection markers may allow the selection of cells carrying the marker, while negative selection markers may allow the selective elimination of cells carrying the marker. Selection markers may confer resistance to drugs or compensate for metabolic or catabolic defects in targeted integrated cells.

In the present application, "antibody" is used in the broadest sense and includes a variety of antibody structures, including but not limited to monoclonal antibodies, polyclonal antibodies, multi-specific antibodies (e.g., bispecific antibodies), half-antibodies, and antibody fragments, provided that the fragments exhibit the desired antigen-binding activity. As used herein, the term "antibody fragment" refers to a molecule other than an intact antibody that includes a portion of the intact antibody that binds to an antigen bound by the intact antibody. Examples of antibody fragments include, but are not limited to, Fv, Fab, Fab', Fab'-SH, F(ab')2; diabodies; linear antibodies; single-chain antibody molecules (e.g., scFv); and multi-specific antibodies formed from antibody fragments. For a review of certain antibody fragments, reference is made to Holliger and Hudson. Nature Biotechnology 23:1126-1136 (2005).

In the present application, the term "targeted integrated cell" refers to a cell into which an exogenous nucleic acid has been introduced, including the progeny of such cell. Targeted integrated cells include "transformants" and "transformed cells", which include primary transformed cells and progeny derived therefrom, regardless of the number of passages. The nucleic acid content of the progeny may not be completely identical to that of the parental cells, but may include mutations. Mutant progeny that has the same function or biological activity as those screened or selected in the originally transformed cell are included herein.

In the present application, the term "vector" refers to a nucleic acid molecule capable of propagating another nucleic acid to which it is connected. The term includes the vector as self-replicating nucleic acid structure and the vector incorporated into the genome of a targeted integrated cell into which it has been introduced. In certain embodiments, a vector directs the expression of a nucleic acid to which it is operably connected. Such vectors are referred to herein as "expression vectors".

As used herein, the term "homologous fragments" refers to sequence fragments that share significant sequence similarity as determined by sequence alignment. For example, two sequence fragments may be about 50%, 60%, 70%, 80%, 90%, 95%, 99%, or 99.9% homologous. Alignment is performed by using algorithms and computer programs (including but not limited to BLAST, FASTA, and HMME), and the alignment compares sequence fragments and calculates the statistical significance of the match based on factors (such as sequence length, sequence identity and similarity, and the presence and length of sequence mismatches and gaps). For example, it may be the ratio of the length of the similar sequence fragment to the length of the aligned region. Homologous sequence fragments may refer to both DNA and protein sequences.

The presently disclosed subject matter provides a targeted integrated cells suitable for an exogenous nucleotide sequence. In some embodiments, the targeted integrated cell includes an exogenous nucleotide sequence integrated at an integration site on the genome of the host cell. The "integration site" includes a nucleic acid sequence within the genome of the targeted integrated cell, where an exogenous nucleotide sequence is inserted into the nucleic acid sequence. In some embodiments, the integration site is between two adjacent nucleotides on the genome of the targeted integrated cell. In some embodiments, the integration site includes a nucleotide stretch, and an exogenous nucleotide sequence may be inserted between any of the nucleotides.

Site-specific integration (SSI), in which a transgene encoding a recombinant protein of interest is integrated at a predetermined genomic site, provides a means to generate more consistent clones and reduces cell line development time. Thus, SSI has emerged as a promising strategy allowing development teams working on host cells (e.g., CHO cell lines) for exogenous expression to repeatedly target the preferred genomic sites thereof, which are capable of exhibiting highly active and stably expressing properties.

A variety of techniques have been used to perform site-specific integration on CHO cell lines, including Cre/Lox recombinase system, Flp/FRT recombinase system, zinc finger nucleases (ZFNs), transcription activator-like effector nucleases (TALENs) and CRISPR/Cas9. Using these techniques, integration efficiency and precision are improved compared to using random knock-in methods. In order to take advantage of recombinase-mediated precise recombination, the target site must be introduced into the cell genome. It may be achieved by simple random integration or targeted mutagenesis.

For site-specific integration methods, the procedures required to obtain the final single-cell clone are lengthy and laborious. In addition, during the integration process, the construct backbone may still be integrated into the genome, resulting in side effects such as gene silencing. Meanwhile, the recombinase system requires two recombination sites to construct RMCE (Recombinase-Mediated Cassette Exchange), which also brings difficulties to the construction of vector cells and the development of subsequent product cells. Zinc finger nucleases (ZFNs), transcription activator-like effector nucleases (TALENs) and CRISPR/Cas9 all use the natural DNA repair mechanism of the cells to integrate payload DNA. However, the frequency of homologous recombination repair decreases as the size of the insertion cassette increases, limiting the quantity of heterologous DNA that may be inserted in single integration.

By using site-specific integration of the target gene, whether it is a fusion protein, a monoclonal antibody or a diabody, the resulting cell line has predictable performance in growth, production and stability compared to the cell line generated by random integration. This method may improve the controllability and repeatability of the integration process, facilitate the stable and high-yield expression of the target gene, and also simplify the subsequent steps such as screening and process development. Therefore, it is highly necessary to develop cells capable of highly-efficiently and stably expressing the target protein.

### First aspect of the present application

Embodiments of the present application provide a nucleic acid, where the nucleic acid includes a nucleic acid fragment as shown in SEQ ID No. 1, and the nucleic acid fragment is used to integrate an exogenous nucleic acid fragment.

The SEQ ID No. 1 is as follows:

TAATTCTGGCAACTATCAAAGGTTCACCAAGGAAGGAATTCTTTATTTGGTATCTGCAAGCG
CCTGGGTGTTCCAATTTCTATCTGCAAATGTTTGTTCCTGCAGACATGTCTAACTATGGTGGAAACAG
AGACCAGCCTCAGATGTCACACTGTGACCTGACCTATAAAAGCTGAACTTTACAAAGCATAAGCTTGG
GTTCATGCCTGAGTGAAACCAGACTGAGTTTCTCTTGTCTCTCCGACTGCTTGCTTGGTCCTGTGCT
GGCTAAAATTTCCATGACAGTAGGGGTGTAAATTATTCAGTTTTAGTTTCCCAGAAAACTAAAAGTAAG
ACTGTCATATGACCTAATGATTTGACTTCTGGGGTCCTTTCAATTAGCTGTTGAAAGGACAATAGATTA
CTCATGGCCATTTAAACAATTCACAGTAGCAAAAAGGTGAG

In one example of the present application, the sequence fragment into which the exogenous nucleic acid fragment is integrated maintains no less than 90% consistency with the nucleic acid fragment as shown in SEQ ID No. 1, for example, the consistency of 90%, 90.5%, 91%, 91.5 %, 92%, 92.5%, 93%, 93.5%, 94%, 94.5%, 95%, 95.5%, 96%, 96.5%, 97%, 97.5%, 98%, 98.5%, 99%, 99.1%, 99.2%, 99.3%, 99.4%, 99.5%, 99.6%, 99.7%, 99.8% and 99.9%.

In the present application, the integration site of the exogenous nucleic acid fragment may correspond to position 1, 2, 3, 4, 5, 6, 7, 8, 9, 10,... 444 of the nucleic acid fragment.

In some examples of the present application, the integration site of the exogenous nucleic acid fragment corresponds to any site within the base interval from positions 11 to 430 of the nucleic acid fragment. Optionally, the integration site of the exogenous nucleic acid fragment corresponds to any site within the base interval from positions 21 to 414 of the nucleic acid fragment. Optionally, the integration site of the exogenous nucleic acid fragment corresponds to any site within the base interval from positions 38 to 402 of the nucleic acid fragment. Optionally, the integration site of the exogenous nucleic acid fragment corresponds to any site within the base interval from positions 53 to 389 of the nucleic acid fragment. Optionally, the integration site of the exogenous nucleic acid fragment corresponds to any site within the base interval from positions 73 to 373 of the nucleic acid fragment. Optionally, the integration site of the exogenous nucleic acid fragment corresponds to any site within the base interval from positions 91 to 360 of the nucleic acid fragment. Optionally, the integration site of the exogenous nucleic acid fragment corresponds to any site within the base interval from positions 108 to 342 of the nucleic acid fragment. Optionally, the integration site of the exogenous nucleic acid fragment corresponds to any site within the base interval from positions 126 to 326 of the nucleic acid fragment. Optionally, the integration site of the exogenous nucleic acid fragment corresponds to any site within the base interval from positions 143 to 310 of the nucleic acid fragment. Optionally, the integration site of the exogenous nucleic acid fragment corresponds to any site within the base interval from positions 160 to 295 of the nucleic acid fragment. Optionally, the integration site of the exogenous nucleic acid fragment corresponds to any site within the base interval from positions 178 to 274 of the nucleic acid fragment. Optionally, the integration site of the exogenous nucleic acid fragment corresponds to any site within the base interval from positions 194 to 263 of the nucleic acid fragment. Optionally, the integration site of the exogenous nucleic acid fragment corresponds to any site within the base interval from positions 209 to 253 of the nucleic acid fragment. Optionally, the integration site of the exogenous nucleic acid fragment corresponds to any site within the base interval from positions 221 to 242 of the nucleic acid fragment. Optionally, the integration site of the exogenous nucleic acid fragment corresponds to any site within the base interval from positions 231 to 240 of the nucleic acid fragment. Optionally, the integration site of the exogenous nucleic acid fragment corresponds to the NW_003616785.1:83044 site in the nucleic acid fragment. Optionally, the integration site of the exogenous nucleic acid fragment corresponds to the NW_003616785.1:83044 site in the nucleic acid fragment in the CHO cell.

In one example of the present application, the exogenous nucleic acid fragment includes a first recombination recognition sequence, a second recombination recognition sequence, a selection marker gene and/or a target gene located between the first recombination recognition sequence and the second recombination recognition sequence, and a promoter that regulates the expression of the selection marker gene and/or the target gene.

In one example of the present application, the exogenous nucleic acid fragment includes a selection marker gene located between the first recombination recognition sequence and the second recombination recognition sequence, and a promoter that regulates the expression of the selection marker gene.

In one example of the present application, the exogenous nucleic acid fragment includes a target gene located between the first recombination recognition sequence and the second recombination recognition sequence, and a promoter that regulates the expression of the target gene.

In one example of the present application, the exogenous nucleic acid fragment includes a selection marker gene and a target gene located between the first recombination recognition sequence and the second recombination recognition sequence, and a promoter that regulates the expression of the selection marker gene and the target gene.

In one example of the present application, the first recombination recognition sequence and the second recombination recognition sequence are generated by a recombinase; the recombinase is a Bxb1 integrase, a ΦC31 integrase, a Cre recombinase or a FLP recombinase.

In one example of the present application, the recombinase is the Bxb1 integrase.

In one example of the present application, the first recombination recognition sequence and the second recombination recognition sequence are each independently selected from one or more of following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxbl attP sequence, a Bxbl attB sequence, an attP sequence, an attB sequence, an attL and an attR sequence.

In one example of the present application, the first recombination recognition sequence and the second recombination recognition sequence are each independently selected from the attR and the attL sequence.

Optionally, the first recombination recognition sequence is the attR, and the second recombination recognition sequence is the attL.

In one example of the present application, the selection marker gene is one or more selected from a neomycin resistance gene, a thymidine kinase gene, a hygromycin phosphotransferase gene, a dihydrofolate reductase gene, a thymidine kinase gene, a glutamine synthetase gene, an asparagine synthetase gene, a tryptophan synthetase gene, a histamine alcohol dehydrogenase gene, an aminoglycoside phosphotransferase gene, a tryptophan synthetase gene and a fluorescent protein gene.

In one example of the present application, the selection marker gene is the fluorescent protein gene.

In one example of the present application, the promoter is a CMV promoter, a SV40 promoter, an RSV promoter, a β-globin promoter, a UBC promoter, an EF1a promoter, a ubiquitin promoter, a β-actin promoter, a PGK1 promoter, a Rosa26 promoter, a HSP70 promoter, a GAPDH promoter, an Eif4A1 promoter, an Egr1 promoter, a FerH promoter, a SM22α promoter or an Endothelin-1 promoter.

In one example of the present application, the target gene encodes one or more of antibodies, recombinant proteins, polypeptides, enzymes, hormones, growth factors and receptors.

In one example of the present application, the target gene encodes the antibody.

In one example of the present application, the nucleotide sequence of the target gene is as shown in SEQ ID No. 2.

In one example of the present application, the nucleotide sequence of the nucleic acid includes the nucleotide sequence as shown in SEQ ID No. 3.

In one example of the present application, the nucleotide sequence of the nucleic acid includes the nucleotide sequence as shown in SEQ ID No. 7.

In one example of the present application, the nucleotide sequence of the nucleic acid includes the nucleotide sequence as shown in SEQ ID No. 9.

### Second aspect of the present application

The embodiments of the present application provide a recombinant vector, where the recombinant vector includes the nucleic acid according to the first aspect, the nucleic acid further includes the first recombination recognition sequence and the second recombination recognition sequence; and the exogenous nucleic acid fragment includes a target gene and/or a selection marker gene.

In one example of the present application, the exogenous nucleic acid fragment is a target gene; the first recombination recognition sequence is a 5' homology arm homologous to the sequence fragment present in the nucleic acid fragment as shown in SEQ ID No. 1; and the second recombination recognition sequence is a 3' homology arm homologous to the sequence fragment present in the nucleic acid fragment as shown in SEQ ID No. 1.

In one example of the present application, the 5' homology arm is the attR and the 3' homology arm is the attL.

In one example of the present application, the exogenous nucleic acid fragment is a selection marker gene; the first recombination recognition sequence is a 5' homology arm homologous to the sequence fragment present in the nucleic acid fragment as shown in SEQ ID No. 1; and the second recombination recognition sequence is a 3' homology arm homologous to the sequence fragment present in the nucleic acid fragment as shown in SEQ ID No. 1.

In one example of the present application, the 5' homology arm is the AttP, and the 3'homology arm is the AttP-GA.

In one example of the present application, the recombinant vector is a lentiviral vector, an adenoviral vector, an adeno-associated viral vector, a herpesvirus vector, a poxvirus vector, a baculovirus vector, a papillomavirus vector, a papovavirus vector, an integrating phage vector, a non-viral vector, a transposon and/or a transposase, an integrase substrate or a plasmid.

In one example of the present application, the recombinant vector is a lentiviral vector, an adenoviral vector, an adeno-associated viral vector, a herpesvirus vector, a poxvirus vector, a baculovirus vector, a papillomavirus vector, a papovavirus vector, an integrating phage vector or a non-viral vector.

### Third aspect of the present application

The embodiments of the present application provide a targeted integrated cell, where the targeted integrated cell includes the nucleic acid according to the first aspect.

In one example of the present application, the targeted integrated cell is a eukaryotic cell;
optionally, the eukaryotic cell is a mammalian cell; and
optionally, the mammalian cell includes a Chinese hamster ovary CHO cell and a human embryonic kidney HEK293 cell.

In one example of the present application, the targeted integrated cell is the Chinese Hamster Ovary CHO cell.

Optionally, the CHO cell includes a CHO host cell, a CHO K1 host cell, a CHO K1SV host cell, a DG44 host cell, DUKXB-11 host cell, a CHOK1S host cell or a CHO K1M host cell.

### Fourth aspect of the present application

The embodiments of the present application provide a preparation method for the targeted integrated cell according to the third aspect, where the preparation method includes the following steps:
introducing the nucleic acid according to the first aspect into a cell, or providing a cell including a nucleic acid fragment as shown in SEQ ID No. 1 and integrating the exogenous nucleic acid fragment into the nucleic acid fragment to prepare the targeted integrated cell.

Optionally, the integration method includes but not limited to the site-specific recombination technique derived from a homologous recombination technique, relying on integrases targeting specific recognition sites to achieve genetic engineering operations such as gene replacement, gene knockout and knock-in between the genome and exogenous DNA, for example, recombinase-mediated cassette exchange, and for example, CRISPR/Cas9-mediated gene targeted integration.

### Fifth aspect of the present application

The embodiments of the present application embodiment provide a method for producing a target gene expression product, where the method includes the following steps: culturing the targeted integrated cell according to the third aspect, and collecting the expression product of the target gene in the exogenous nucleic acid fragment.

### Sixth aspect of the present application

The embodiments of the present application provide a use of the nucleic acid according to the first aspect in preparing a protein or a polypeptide, where the protein or the polypeptide is expressed by the exogenous nucleic acid fragment.

The targeted integrated cell in the present application has stable and high-yielding characteristics.

In the present application, the nucleotide sequence of the integration site and/or flanking the integration site may be identified through experiments. In some embodiments of the present application, the nucleotide sequence of the integration site and/or flanking the integration site may be identified by a whole-genome screening method to isolate the host cell; in some embodiments of the present application, the nucleotide sequence of the integration site and/or flanking the integration site may be identified by using the whole-genome screening method after a transposase based cassette integration event; in some embodiments of the present application, the nucleotide sequence of the integration site and/or flanking the integration site may be identified by brute force random integration screening; in some embodiments of the present application, the nucleotide sequence of the integration site and/or flanking the integration site may be determined by a conventional sequencing method (such as targeted locus amplification) followed by next-generation sequencing and whole-genome; and in some embodiments of the present application, the position of the integration site on the chromosome may be determined by a conventional cell biology method (such as fluorescence in situ hybridization analysis).

The embodiments of the present application will be described below in detail in conjunction with the examples. It should be understood that these examples are only used to illustrate the present application and are not intended to limit the scope of the present application. For experimental methods, the specific conditions of which are not specified in the following examples, priority shall be given to the guidance provided in the present application; they may also be in accordance with experimental manuals or conventional conditions in the art, may also be conditions as recommended by manufacturers, or refers to experimental methods known in the art.

In the specific examples described below, with respect to the measurement parameters of raw material components, unless otherwise specified, there may be slight deviations within the range of weighing accuracy. With respect to temperature and time parameters, acceptable deviations caused by instrument testing precision or operational precision are permitted.

The consumables in the following specific examples involve: Neon Resuspension Buffer R (ThermoFisher), which is a resuspension buffer dedicated for cell electroporation instrument; E1 Buffer (ThermoFisher), which is an electroporation buffer; a recovery medium, which is prepared by adding 1% GlutaMAX (ThermoFisher) to 80% (v/v) EX-CELL CHO Cloning Medium (Sigma-Aldrich) and 20% (v/v) EX-CELL Advanced CHO Fed-batch Medium (Sigma-Aldrich); an expansion medium and a passage medium, which are both prepared by adding 1% GlutaMAX (ThermoFisher) to EX-CELL Advanced CHO Fed-batch Medium; a pressurized medium 1, which is prepared by adding G418 to the passage medium to a final concentration of 200µg/ml; a pressurized medium 2, which is prepared by adding Hygromycin to the passage medium to a final concentration of 200µg/ml; a conditioned medium, which is the supernatant obtained by inoculating CHO-K1 in the passage medium, culturing for 1 day, and then performing sterile filtration; a cloning medium, which is prepared by adding 1% GlutaMAX to 75% (v/v) EX-CELL CHO Cloning Medium, 20% (v/v) conditioned medium, and 5% (v/v) ClonaCell-CHO ACF Supplement; and in fed-batch medium, basal medium which is prepared by adding 1% GlutaMAX (ThermoFisher) to EX-CELL Advanced CHO Fed-batch Medium, and the feed medium, which is Cell Boost 7a/7b (HyClone).

### Example 1

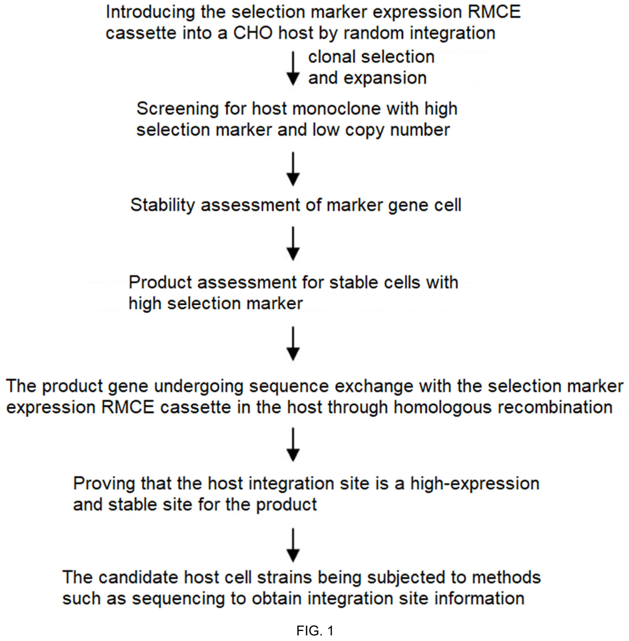

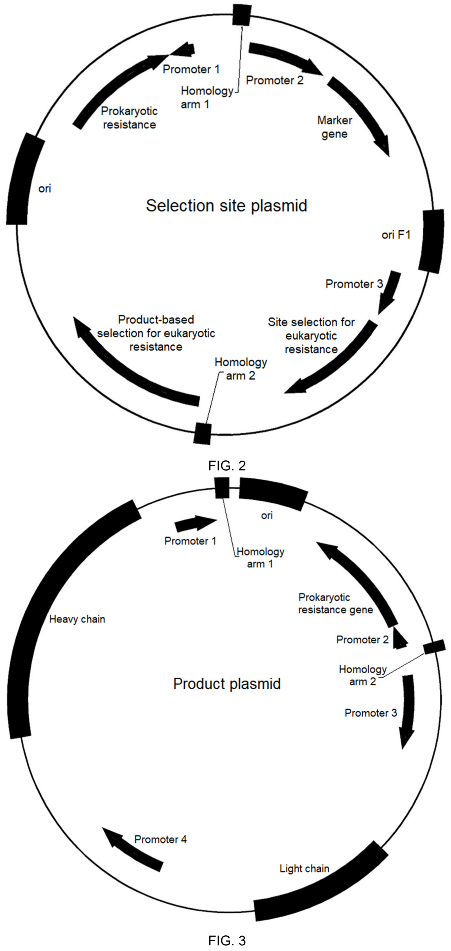

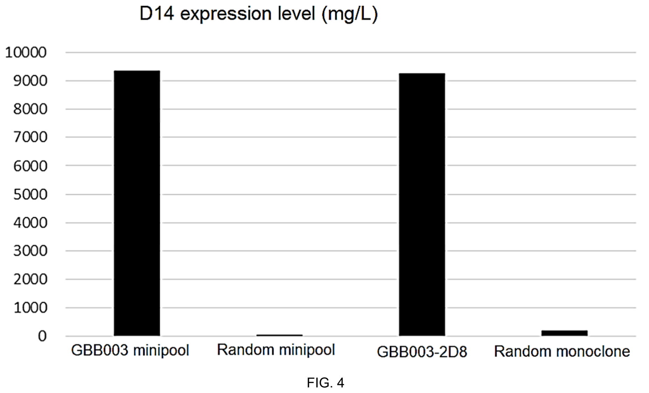

The operation flow of this example, as shown in FIG. 1, mainly included the following steps:
(1) constructing a recombinant plasmid containing RMCE by using the green fluorescent protein gene (EGFP) as a marker gene for screening and using the attp sequence as a homology arm, as shown in FIG. 2;
(2) purifying and recovering a linear DNA after linearizing the constructed plasmid;
(3) collecting 3×10⁶ CHO cells into a 50mL centrifuge tube, centrifuging at 1,000rpm for 5 min at a room temperature, and discarding the supernatant;
(4) resuspending the cells with 100µL of R Buffer, a resuspension buffer dedicated for a cell electroporation instrument;
(5) adding 15µg of the linearized plasmid in step (2) to the resuspended cells in step (4), mixing gently, and pipetting 50 times to avoid generating air bubbles;
(6) turning on the cell electroporation instrument, adjusting the parameters, installing an electroporation tank into the electroporation instrument, and adding 3mL of electroporation E1 Buffer into the tank;
(7) sucking the cell plasmid suspension in step (5) into the tip of the electroporation gun, loading into the electroporation tank, and performing electroporation;
(8) immediately transferring the electroporated cells into a 6-well plate added with 2mL of the recovery medium, and placing in a 37°C, 5% CO₂ incubator, and standing overnight for culturing;
(9) pressurizing by using the pressurized medium 1, after electroporation for 24 h, and diluting and plating into 480 wells;
(10) obtaining a stable fluorescent pool after culturing for about 2 weeks, expanding the pool with higher overall fluorescence, and then continuing culturing until the cell doubling time is less than 24 h and the survival rate is greater than 95%;
(11) diluting the recovered cells limitedly and culturing by using the cloning medium to complete clonal selection (monoclonalization);
(12) expanding and culturing, after 14 days, the fluorescent monoclonal cells in the expansion medium to form cell strains, monitoring the growth curve and detecting the fluorescence through the fed-batch medium and the feed medium in the shake flask stage, and retaining the cell strains with a good growth curve and a high fluorescence and recording as GBB003 cells.
(13) performing an approximately 90-day passage stability study on the high-fluorescence cell strains above by using the passage medium to confirm that the GBB003 cells with small fluorescence value changes and stable cell strain growth were stable high-fluorescence cells;
(14) cloning the target gene 1 fragment, the target gene 2 fragment, and the target gene 3 fragment according to step (1) into different plasmids to construct three recombinant plasmids containing RMCE, as in FIG. 3,
   where the target gene fragment 1 had the sequence as shown in SEQ ID No. 2, and the SEQ ID No. 2 was as follows: 
TCAACACTCGCCTCTATTGAAGCTCTTGGTCACGGGTGAAGACAGGCCCTGGTGGGTCA
CCTCGCAGGCGTACACTTTGTGCTTCTCGTAGTCGGCCTTGGACAGGGTCAGGGTAGAGGACAGG
GAGTAGGTGGAGTCCTTAGAGTCCTGTTCTGTCACGGACTCTTGAGAGTTGCCAGACTGCAGGGCG
TTGTCGACCTTCCACTGCACCTTGGCCTCTCTAGGGTAGAAGTTGTTCAGCAGGCACACAACGGAG
GCGGTGCCAGATTTCAGCTGCTCGTCGGAAGGAGGAAAGATGAACACAGAAGGAGCGGCCACGGT
CCGCTTGATTTCCAGCTTGGTTCCCTGGCCGAAGGTGTAGGGGGTCTGCAGAGCCTGCATGCAGTA
GTAAAAGCCCACGTCCTCAGCTTCCACTCTGCTGATCTTCAGTGTGAAGTCGGTGCCAGATCCGGA
GCCGGAGAATCTATCGGGCACTCCGCTGGCCCGGTTGGAGCCCAGGTAGATCAGCAGCTGAGGGG
ATTGGCCGCTCTTCTGCAGGTACCAATCCAGGTAGTTGTAGCCGATGGAATACAGGAGGCTCTGGGA
GGACCGGCAAGAGATAGAAGCAGGTTCGCCAGGGGTGACAGGCAGAGAAAGTGGGGACTGTGTCA
TCACGATATCACATCTAGCGCCTCTCAGCCACAGCAGGAGCAGGCCCAGCAGCTGAGCAGGCACTC
TCATGTCCATGGTGGCGGCAAGCTTCGTGTCAAGGACGGTGACTGCAGAAAAGACCCATGGAAAGG
AACAGTCTGTTAGTCTGTCAGCTATTATGTCTGGTGGCGCGCGCGGCAGCAACGAGTACTGCTCAGA
CTACACTGCCCTCCACCGTTAACAGCACCGCAACGGGAGTTACCTCTGACTCTTATCAGAACACAAC
AACTCAGCTGCCTGCATCTTCTTCTGCCGCTGCCTTAAGTCTTCCAAATGCGTCAGCGGTGCAAGCC
CGCTCCCCGAGCTCATTTTCAGACACATACCCTACCGCCACGGCCTTGTGCGGCACACTGGTGGTG
GTGGGCATCGTGCTGTGCCTAAGTCTGGCCTCCACTGTTAGGAGCAAGGAGCTGCCGAGCGACCAT
GAGTCGCTGGAGGCATGGGAGCAGGGCTCGGATGTAGAAGCTCCGCCGCTACCGGAGAAGAGCCC
ATGTCCGGAACACGTACCCGAGATTCGCGTGGAGATCCCACGTTATGTTTAATAAAAACTGCGGGCA
CTGGGGACGGTGGTGTTGTATATGTGAATTTGTAAATAATAAATGAGACCCCATCCTGTAAAAATACAG
AGTCCGTGTCAGTCTCTGAAGGACAGTGTATTGGCATATAGCCAATAAAGAGAGTTGTGGCAAAGAG
CCATGTTATGGATTAGTAATGGAAAGTATCGTCACCAATAGGGGAGTGGTCAATAATGGTCAATAACCC
ACACCTATAGGCTAAGCTATACCATCACCTATAACATGAGGAAGCGGGGGTGTATAGACCCCAAGCCA
AAAACAGTATAGCATGCATAAGAAGCCAAGGGGGTGGGCCTATAGACTCTATAGGCGGTACTTACGTC
ACTCTTGGCACGGGGAATCCGCGTTCCAATGCACCGTTCCCGGCCGCGGAGGCTGGATCGGTCCC
GGTGTCTTCTATGGAGGTCAAAACAGCGTGGATGGCGTCTCCAGGCGATCTGACGGTTCACTAAAC
GAGCTCTGCTTATATAGACCTCCCACCGTACACGCCTACCGCCCATTTGCGTCAATGGGGCGGAGTT
GTTACGACATTTTGGAAAGTCCCGTTGATTTTGGTGCCAAAACAAACTCCCATTGACGTCAATGGGGT
 
GGAGACTTGGAAATCCCCGTGAGTCAAACCGCTATCCACGCCCATTGATGTACTGCCAAAACCGCAT
CACCATGGTAATAGCGATGACTAATACGTAGATGTACTGCCAAGTAGGAAAGTCCCATAAGGTCATGTA
CTGGGCATAATGCCAGGCGGGCCATTTACCGTCATTGACGTCAATAGGGGGCGTACTTGGCATATGA
TACACTTGATGTACTGCCAAGTGGGCAGTTTACCGTAAATACTCCACCCATTGACGTCAATGGAAAGT
CCCTATTGGCGTTACTATGGGAACATACGTCATTATTGACGTCAATGGGCGGGGGTCGTTGGGCGGT
CAGCCAGGCGGGCCATTTACCGTAAGTTATGTAACGCGGAACTCCATATATGGGCTATGAACTAATGA
CCCCGTAATTGATTACTATTAATAACTAGTCAATAATCAATGTCAACATGGCGGTAATGTTGGACATGAG
CCAATATAAATGTACATATTATGATATGGCGGCC
(15) selecting the stable high-fluorescence cells GBB003 in step (13) for transfection and integration verification, and amplifying and linearizing the recombinant plasmids in the above step (14), and then co-transfecting with Bxb-1 integrase into the stable high-fluorescence cells GBB003, where the transfection step was as described above;
(16) constructing a minipool from cell population after one day transfection, and performing pressurized screening by using the pressurized medium 2;
(17) expanding, after 7-10 days, the non-fluorescent cells in the minipool and diluting limitedly for clonal selection;
(18) placing and culturing the non-fluorescent cells in the monoclonal cells in a constant temperature incubator (37°C, 80% humidity) for further expansion, and culturing in the shake flask stage after expanding, where after approximately 1 month, the cell strains were subjected to fed-batch culture (Advance+1%Glutamax, i.e. the fed-batch medium, for the first three days), 3% (v/v) Cell Boost 7a and 0.3% (v/v) Cell Boost 7b (cytiva) were added at the same time on days 3 and 5, 5% (v/v) Cell Boost 7a and 0.5 % (v/v) Cell Boost 7b (cytiva) were added at the same time on each of the days 7, 9, 11, and 13, the sugar was replenished according to the sugar consumption of cells and the expression level was evaluated on days 3, 5, 7, 9, 11, and 13, and the comparison results of the expression levels of the products of site specific integration, minipool cell pool (GBB003-minipool) and single-copy cell strain (GBB003-2D8), and the minipool cell pool (random minipool) and single-copy cell strain (random monoclonal) of the random integration cultured under the same conditions were shown in FIG. 4.

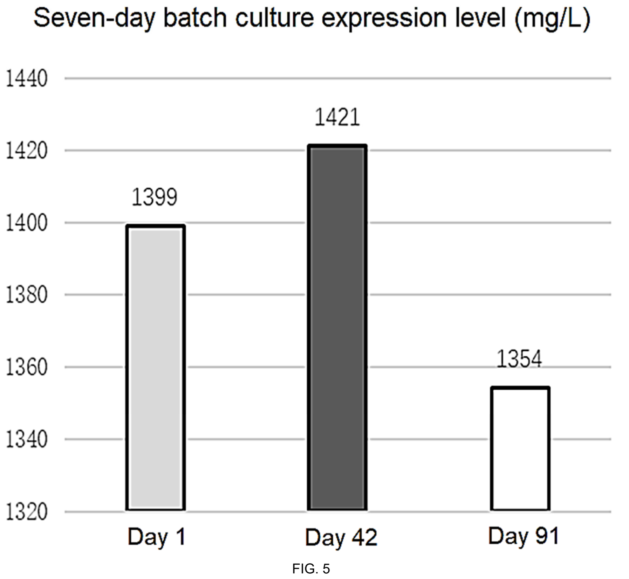

In order to investigate the stability of cell clones with targeted integration of the target gene 1, the cloned cells were continuously passaged for approximately 90 days for stability assessment, P1, P13, and P26 were each batch cultured for 7 days, and sugar was replenished on the 3rd, 5th, and 7th days according to the sugar consumption of the cells. The expression levels (FIG. 5) showed that the monoclonal cells were stable and has high yield. The second-generation whole genome sequencing was performed on the cell strain GBB003 which can highly express both the fluorescent gene and the target gene, and the acquired annotation information for the integration site in the CHO was as follows: NW_003616785.1:83044. When the fluorescent gene was inserted at the position of NW_003616785.1:83044 of the cell strain GBB003, the fluorescent gene was highly expressed by the cell strain GBB003. When the target gene was inserted at the position of NW_003616785.1:83044 of the cell strain GBB003, the target gene was highly expressed by the cell strain GBB003.

The sequence fragment after integrating the target gene 1 was as shown in SEQ ID No. 3, and the SEQID No.3 was as follows:

CAAGTAAAACCTCTACAAATGTGGTATGGCTGATTAGCCGGCTATCGTACTCGAGTCAACA
CTCGCCTCTATTGAAGCTCTTGGTCACGGGTGAAGACAGGCCCTGGTGGGTCACCTCGCAGGCGTA
CACTTTGTGCTTCTCGTAGTCGGCCTTGGACAGGGTCAGGGTAGAGGACAGGGAGTAGGTGGAGT
CCTTAGAGTCCTGTTCTGTCACGGACTCTTGAGAGTTGCCAGACTGCAGGGCGTTGTCGACCTTCC
ACTGCACCTTGGCCTCTCTAGGGTAGAAGTTGTTCAGCAGGCACACAACGGAGGCGGTGCCAGATT
TCAGCTGCTCGTCGGAAGGAGGAAAGATGAACACAGAAGGAGCGGCCACGGTCCGCTTGATTTCC
AGCTTGGTTCCCTGGCCGAAGGTGTAGGGGGTCTGCAGAGCCTGCATGCAGTAGTAAAAGCCCACG
TCCTCAGCTTCCACTCTGCTGATCTTCAGTGTGAAGTCGGTGCCAGATCCGGAGCCGGAGAATCTAT
CGGGCACTCCGCTGGCCCGGTTGGAGCCCAGGTAGATCAGCAGCTGAGGGGATTGGCCGCTCTTC
TGCAGGTACCAATCCAGGTAGTTGTAGCCGATGGAATACAGGAGGCTCTGGGAGGACCGGCAAGAG
ATAGAAGCAGGTTCGCCAGGGGTGACAGGCAGAGAAAGTGGGGACTGTGTCATCACGATATCACAT
CTAGCGCCTCTCAGCCACAGCAGGAGCAGGCCCAGCAGCTGAGCAGGCACTCTCATGTCCATGGT
GGCGGCAAGCTTCGTGTCAAGGACGGTGACTGCAGAAAAGACCCATGGAAAGGAACAGTCTGTTAG
TCTGTCAGCTATTATGTCTGGTGGCGCGCGCGGCAGCAACGAGTACTGCTCAGACTACACTGCCCT
CCACCGTTAACAGCACCGCAACGGGAGTTACCTCTGACTCTTATCAGAACACAACAACTCAGCTGCC
TGCATCTTCTTCTGCCGCTGCCTTAAGTCTTCCAAATGCGTCAGCGGTGCAAGCCCGCTCCCCGAG
CTCATTTTCAGACACATACCCTACCGCCACGGCCTTGTGCGGCACACTGGTGGTGGTGGGCATCGT
GCTGTGCCTAAGTCTGGCCTCCACTGTTAGGAGCAAGGAGCTGCCGAGCGACCATGAGTCGCTGG
AGGCATGGGAGCAGGGCTCGGATGTAGAAGCTCCGCCGCTACCGGAGAAGAGCCCATGTCCGGAA
CACGTACCCGAGATTCGCGTGGAGATCCCACGTTATGTTTAATAAAAACTGCGGGCACTGGGGACGG

TGGTGTTGTATATGTGAATTTGTAAATAATAAATGAGACCCCATCCTGTAAAAATACAGAGTCCGTGTC
AGTCTCTGAAGGACAGTGTATTGGCATATAGCCAATAAAGAGAGTTGTGGCAAAGAGCCATGTTATGG
ATTAGTAATGGAAAGTATCGTCACCAATAGGGGAGTGGTCAATAATGGTCAATAACCCACACCTATAGG
CTAAGCTATACCATCACCTATAACATGAGGAAGCGGGGGTGTATAGACCCCAAGCCAAAAACAGTATA
GCATGCATAAGAAGCCAAGGGGGTGGGCCTATAGACTCTATAGGCGGTACTTACGTCACTCTTGGCA
CGGGGAATCCGCGTTCCAATGCACCGTTCCCGGCCGCGGAGGCTGGATCGGTCCCGGTGTCTTCT
ATGGAGGTCAAAACAGCGTGGATGGCGTCTCCAGGCGATCTGACGGTTCACTAAACGAGCTCTGCT
TATATAGACCTCCCACCGTACACGCCTACCGCCCATTTGCGTCAATGGGGCGGAGTTGTTACGACATT
TTGGAAAGTCCCGTTGATTTTGGTGCCAAAACAAACTCCCATTGACGTCAATGGGGTGGAGACTTGG
AAATCCCCGTGAGTCAAACCGCTATCCACGCCCATTGATGTACTGCCAAAACCGCATCACCATGGTA
ATAGCGATGACTAATACGTAGATGTACTGCCAAGTAGGAAAGTCCCATAAGGTCATGTACTGGGCATA
ATGCCAGGCGGGCCATTTACCGTCATTGACGTCAATAGGGGGCGTACTTGGCATATGATACACTTGAT
GTACTGCCAAGTGGGCAGTTTACCGTAAATACTCCACCCATTGACGTCAATGGAAAGTCCCTATTGGC
GTTACTATGGGAACATACGTCATTATTGACGTCAATGGGCGGGGGTCGTTGGGCGGTCAGCCAGGC
GGGCCATTTACCGTAAGTTATGTAACGCGGAACTCCATATATGGGCTATGAACTAATGACCCCGTAATT
GATTACTATTAATAACTAGTCAATAATCAATGTCAACATGGCGGTAATGTTGGACATGAGCCAATATAAA
TGTACATATTATGATATGGCGGCCGCATGATCCTGACGACGGAGACCGCGGTGGTTGACCAGACAAA
CCTACATCGCGAAGCAGCGCAAAACGCCTAACCCTAAGCAGATTCTTCATGCAATTGTCGGTCAAGC
CTTGCCTTGTTGTAGCTTAAATTTTGCTCGCGCACTACTCAGCGACCTCCAACACACAAGCAGGGAG
CAGATACTGGCTTAACTATGCGGCATCAGAGCAGATTGTACTGAGAGTCGACCATAGGGGATCGGGA
GATCTCCCGATCCGTCGACGTCAGGTGGCACTTTTCGGGGAAATGTGCGCGGAACCCCTATTTGTTT
ATTTTTCTAAATACATTCAAATATGTATCCGCTCATGAGACAATAACCCTGATAAATGCTTCAATAATATT
GAAAAAGGAAGAGTATGAGTATTCAACATTTCCGTGTCGCCCTTATTCCCTTTTTTGCGGCATTTTGC
CTTCCTGTTTTTGCTCACCCAGAAACGCTGGTGAAAGTAAAAGATGCTGAAGATCAGTTGGGTGCAC
GAGTGGGTTACATCGAACTGGATCTCAACAGCGGTAAGATCCTTGAGAGTTTTCGCCCCGAAGAACG
TTTTCCAATGATGAGCACTTTTAAAGTTCTGCTATGTGGCGCGGTATTATCCCGTATTGACGCCGGGC
AAGAGCAACTCGGTCGCCGCATACACTATTCTCAGAATGACTTGGTTGAGTACTCACCAGTCACAGA
AAAGCATCTTACGGATGGCATGACAGTAAGAGAATTATGCAGTGCTGCCATAACCATGAGTGATAACA
CTGCGGCCAACTTACTTCTGACAACGATCGGAGGACCGAAGGAGCTAACCGCTTTTTTGCACAACAT
GGGGGATCATGTAACTCGCCTTGATCGTTGGGAACCGGAGCTGAATGAAGCCATACCAAACGACGA

GCGTGACACCACGATGCCTGTAGCAATGGCAACAACGTTGCGCAAACTATTAACTGGCGAACTACTT
ACTCTAGCTTCCCGGCAACAATTAATAGACTGGATGGAGGCGGATAAAGTTGCAGGACCACTTCTGC
GCTCGGCCCTTCCGGCTGGCTGGTTTATTGCTGATAAATCTGGAGCCGGTGAGCGTGGGTCTCGCG
GTATCATTGCAGCACTGGGGCCAGATGGTAAGCCCTCCCGTATCGTAGTTATCTACAAGAGAAACTCA
GTCTGGTTTCACTCAGGCATGAACCCAAGCTTATGCTTTGTAAAGTTCAGCTTTTATAGGTCAGGTCA
CAGTGTGACATCTGAGGCTGGTCTCTGTTTCCACCATAGTTAGACATGTCTGCAGGAACAAACATTTG
CAGATAGAAATTGGAACACCCAGGCGCTTGCAGATACCAAATAAAGAATTCCTTCCTTGGTGAACCTT
TGATAGTTGCCAGAATTATTCCTTAAGGTAGTTTATGTGAATGTTTGCCTTCCCAAGACAAGAGGAGG
GGGGCAGAGTGAACTCAGCACATGAGTAAAACAGTAACTCACTCTGTTTTCTAAGCAATCCTACAGTT
TCCAGGCAGGAAATTCTATTAGACAATCAAAACACAGAGTAATAGTTTGATTTTATTAGGTATGTAATAT
GTAAGGGCATATATATTTCATCACCACTGCCAATGGCAGTTCTACTATGTGTATGATCTTATTTCTTAATT
ATTTAATTTTTCTGTGTCTTCAACACAACTCTACTAGACG

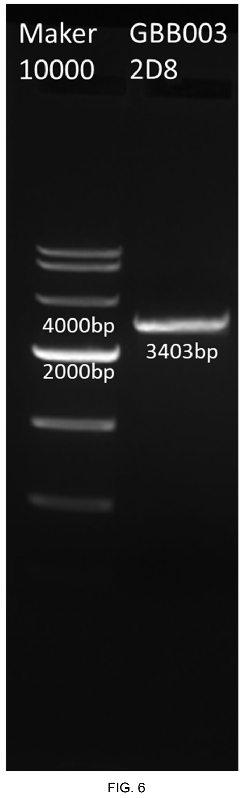

The genome of the monoclonal cell strain was amplified using the above-mentioned breakpoint forward primer F1 (SEQ ID No. 4): AGACCAGCCTCAGATGTCACAC and the reverse primer R1 (SEQ ID No. 5): AGGCACACAACGGAGGCGGT of the target gene on the plasmid used for integration, to obtain a fragment consistent with the theoretical size of 3403 bp, as shown in FIG. 6 (band corresponding to lane GBB003-2D8).

In the above, the sequence fragment after integrating the target gene 1 included attL, the nucleotide sequence of attL was as shown in SEQ ID No. 6, SEQ ID No. 6: ATGATCCTGACGACGGAGACCGCGGTGGTTGACCAGACAAACC. In the sequence fragment after integrating the target gene 1, the part before attL was the exogenous sequence, and the part after attL was the endogenous sequence. The exogenous sequence included the target gene 1. The target gene 1 may encode a monoclonal antibody.

The amplified band was cut and recovered from the gel, and purified, and multiple pairs of primers were designed for DNA sequencing. After splicing the sequencing results, the sequence was as shown in SEQ ID No. 3, which was consistent with the theoretical sequence (sequence SEQ ID No. 3), thus confirming that the target gene was correctly integrated into the target site.

Similarly, after integrating the target gene 2, the sequence fragment of the cell strain capable of highly expressing the target gene was as shown in SEQ ID No. 7, and the SEQ ID No. 7 was as follows:

TGGGTCACCTCGCAGGCGTACACCTTGTGCTTCTCGTAGTCGGCCTTGCTGAGGGTCAG

GGTAGAGGACAGGCTGTAGGTGGAGTCCTTAGAATCCTGCTCGGTGACAGACTCTTGGGAGTTGCC
GGACTGCAGGGCGTTGTCCACCTTCCACTGCACTTTGGCCTCTCTAGGGTAGAAGTTGTTCAGCAG
GCACACTACAGAAGCGGTTCCGCTCTTCAGTTGTTCGTCAGAAGGAGGGAAGATGAACACGGAAGG
AGCGGCCACGGTCCGCTTGATTTCCACTTTGGTGCCTCCGCCGAAGGTCAGAGGCAGGTCCCGAG
AATGCTGGCAGTAGTACACGGCAAAATCCTCGGGTTCCAGGGAGGAGATTGTCAGGGTGAAGTCGG
TGCCAGAGCCGCTACCGGAGAATCTGGCTGGCACGCCGGATTCCAGGTAAGAGGCCAGGTAGATC
AGCAGCCGAGGGGCCTGGCCAGGCTTCTGCTGGTACCAGTGCAGATAGGAGTAGCCAGAAGTGGA
CACGCCCTTGGAGGCTCTACAGGACAGTGTAGCCCTTTCGCCGGGGCTCAGAGACAGGGTAGCAG
GAGACTGGGTCAGCACGATCTCGGCTCTGCCAGCCCACAGTAGTGGTAGTAGTAGTAGTAGTGCTC
GCATGGTGGCGGCAAGCTTCGTGTCAAGGACGGTGACTGCAGAAAAGACCCATGGAAAGGAACAG
TCTGTTAGTCTGTCAGCTATTATGTCTGGTGGCGCGCGCGGCAGCAACGAGTACTGCTCAGACTACA
CTGCCCTCCACCGTTAACAGCACCGCAACGGGAGTTACCTCTGACTCTTATCAGAACACAACAACTC
AGCTGCCTGCATCTTCTTCTGCCGCTGCCTTAAGTCTTCCAAATGCGTCAGCGGTGCAAGCCCGCT
CCCCGAGCTCATTTTCAGACACATACCCTACCGCCACGGCCTTGTGCGGCACACTGGTGGTGGTGG
GCATCGTGCTGTGCCTAAGTCTGGCCTCCACTGTTAGGAGCAAGGAGCTGCCGAGCGACCATGAGT
CGCTGGAGGCATGGGAGCAGGGCTCGGATGTAGAAGCTCCGCCGCTACCGGAGAAGAGCCCATGT
CCGGAACACGTACCCGAGATTCGCGTGGAGATCCCACGTTATGTTTAATAAAAACTGCGGGCACTGG
GGACGGTGGTGTTGTATATGTGAATTTGTAAATAATAAATGAGACCCCATCCTGTAAAAATACAGAGTC
CGTGTCAGTCTCTGAAGGACAGTGTATTGGCATATAGCCAATAAAGAGAGTTGTGGCAAAGAGCCAT
GTTATGGATTAGTAATGGAAAGTATCGTCACCAATAGGGGAGTGGTCAATAATGGTCAATAACCCACA
CCTATAGGCTAAGCTATACCATCACCTATAACATGAGGAAGCGGGGGTGTATAGACCCCAAGCCAAAA
ACAGTATAGCATGCATAAGAAGCCAAGGGGGTGGGCCTATAGACTCTATAGGCGGTACTTACGTCACT
CTTGGCACGGGGAATCCGCGTTCCAATGCACCGTTCCCGGCCGCGGAGGCTGGATCGGTCCCGGT
GTCTTCTATGGAGGTCAAAACAGCGTGGATGGCGTCTCCAGGCGATCTGACGGTTCACTAAACGAG
CTCTGCTTATATAGACCTCCCACCGTACACGCCTACCGCCCATTTGCGTCAATGGGGCGGAGTTGTT
ACGACATTTTGGAAAGTCCCGTTGATTTTGGTGCCAAAACAAACTCCCATTGACGTCAATGGGGTGG
AGACTTGGAAATCCCCGTGAGTCAAACCGCTATCCACGCCCATTGATGTACTGCCAAAACCGCATCA
CCATGGTAATAGCGATGACTAATACGTAGATGTACTGCCAAGTAGGAAAGTCCCATAAGGTCATGTACT
GGGCATAATGCCAGGCGGGCCATTTACCGTCATTGACGTCAATAGGGGGCGTACTTGGCATATGATA
CACTTGATGTACTGCCAAGTGGGCAGTTTACCGTAAATACTCCACCCATTGACGTCAATGGAAAGTCC

CTATTGGCGTTACTATGGGAACATACGTCATTATTGACGTCAATGGGCGGGGGTCGTTGGGCGGTCA
GCCAGGCGGGCCATTTACCGTAAGTTATGTAACGCGGAACTCCATATATGGGCTATGAACTAATGACC
CCGTAATTGATTACTATTAATAACTAGTCAATAATCAATGTCAACATGGCGGTAATGTTGGACATGAGCC
AATATAAATGTACATATTATGATATGGCGGCCGCATGATCCTGACGACGGAGACCGCGGTGGTTGACC
AGACAAACCTACATCGCGAAGCAGCGCAAAACGCCTAACCCTAAGCAGATTCTTCATGCAATTGTCG
GTCAAGCCTTGCCTTGTTGTAGCTTAAATTTTGCTCGCGCACTACTCAGCGACCTCCAACACACAAG
CAGGGAGCAGATACTGGCTTAACTATGCGGCATCAGAGCAGATTGTACTGAGAGTCGACCATAGGGG
ATCGGGAGATCTCCCGATCCGTCGACGTCAGGTGGCACTTTTCGGGGAAATGTGCGCGGAACCCCT
ATTTGTTTATTTTTCTAAATACATTCAAATATGTATCCGCTCATGAGACAATAACCCTGATAAATGCTTCA
ATAATATTGAAAAAGGAAGAGTATGAGTATTCAACATTTCCGTGTCGCCCTTATTCCCTTTTTTGCGGC
ATTTTGCCTTCCTGTTTTTGCTCACCCAGAAACGCTGGTGAAAGTAAAAGATGCTGAAGATCAGTTGG
GTGCACGAGTGGGTTACATCGAACTGGATCTCAACAGCGGTAAGATCCTTGAGAGTTTTCGCCCCGA
AGAACGTTTTCCAATGATGAGCACTTTTAAAGTTCTGCTATGTGGCGCGGTATTATCCCGTATTGACG
CCGGGCAAGAGCAACTCGGTCGCCGCATACACTATTCTCAGAATGACTTGGTTGAGTACTCACCAGT
CACAGAAAAGCATCTTACGGATGGCATGACAGTAAGAGAATTATGCAGTGCTGCCATAACCATGAGTG
ATAACACTGCGGCCAACTTACTTCTGACAACGATCGGAGGACCGAAGGAGCTAACCGCTTTTTTGCA
CAACATGGGGGATCATGTAACTCGCCTTGATCGTTGGGAACCGGAGCTGAATGAAGCCATACCAAAC
GACGAGCGTGACACCACGATGCCTGTAGCAATGGCAACAACGTTGCGCAAACTATTAACTGGCGAA
CTACTTACTCTAGCTTCCCGGCAACAATTAATAGACTGGATGGAGGCGGATAAAGTTGCAGGACCACT
TCTGCGCTCGGCCCTTCCGGCTGGCTGGTTTATTGCTGATAAATCTGGAGCCGGTGAGCGTGGGTC
TCGCGGTATCATTGCAGCACTGGGGCCAGATGGTAAGCCCTCCCGTATCGTAGTTATCTACAAGAGA
AACTCAGTCTGGTTTCACTCAGGCATGAACCCAAGCTTATGCTTTGTAAAGTTCAGCTTTTATAGGTC
AGGTCACAGTGTGACATCTGAGGCTGGTCTCTGTTTCCACCATAGTTAGACATGTCTGCAGGAACAA
ACATTTGCAGATAGAAATTGGAACACCCAGGCGCTTGCAGATACCAAATAAAGAATTCCTTCC

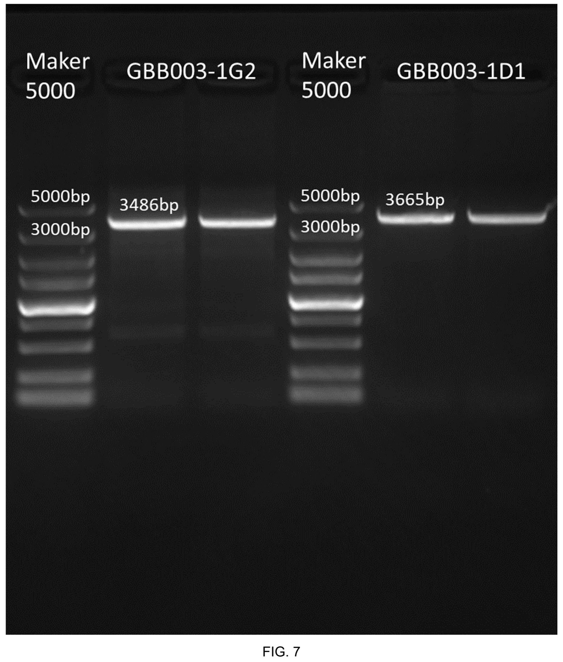

The genome of the monoclonal cell strain was amplified using the above-mentioned breakpoint forward primer F1 (SEQ ID No. 4): AGACCAGCCTCAGATGTCACAC and the reverse primer R2 (SEQ ID No. 8): CCTTAGAATCCTGCTCGGTGA of the product gene on the plasmid used for the product to obtain a fragment consistent with the theoretical size (3486 bp), as shown in FIG. 7 (band corresponding to lane GBB003-1G2).

The amplified band was cut and recovered from the gel and purified, and multiple pairs of primers were designed for DNA sequencing. After splicing the sequencing results, the sequence was as shown in SEQ ID No. 7, which was consistent with the theoretical sequence (sequence SEQ ID No. 7), thus confirming that the target gene was correctly integrated into the target site. In the above, the sequence fragment after integrating the target gene 2 included the attL. In the sequence fragment after integrating the target gene 2, the part before the attL was the exogenous sequence, and the part thereafter was the endogenous sequence. The exogenous sequence included the target gene 2. After integrating the target gene 2 into the cell strain with high expression of the target gene, the marker gene EGFP was replaced by the fragment where the target gene 2 was located.

Similarly, the sequence fragment of the cell strain with high expression of the target gene after integrating the target gene 3 was as shown in SEQ ID No. 9, and the SEQ ID No. 9 was as follows:

GGTGGCGGTGGCAAGCTTCGTGTCAAGGACGGTGACTGCAGAAAAGACCCATGGAAAG
GAACAGTCTGTTAGTCTGTCAGCTATTATGTCTGGTGGCGCGCGCGGCAGCAACGAGTACTGCTCA
GACTACACTGCCCTCCACCGTTAACAGCACCGCAACGGGAGTTACCTCTGACTCTTATCAGAACACA
ACAACTCAGCTGCCTGCATCTTCTTCTGCCGCTGCCTTAAGTCTTCCAAATGCGTCAGCGGTGCAAG
CCCGCTCCCCGAGCTCATTTTCAGACACATACCCTACCGCCACGGCCTTGTGCGGCACACTGGTGG
TGGTGGGCATCGTGCTGTGCCTAAGTCTGGCCTCCACTGTTAGGAGCAAGGAGCTGCCGAGCGAC
CATGAGTCGCTGGAGGCATGGGAGCAGGGCTCGGATGTAGAAGCTCCGCCGCTACCGGAGAAGAG
CCCATGTCCGGAACACGTACCCGAGATTCGCGTGGAGATCCCACGTTATGTTTAATAAAAACTGCGG
GCACTGGGGACGGTGGTGTTGTATATGTGAATTTGTAAATAATAAATGAGACCCCATCCTGTAAAAATA
CAGAGTCCGTGTCAGTCTCTGAAGGACAGTGTATTGGCATATAGCCAATAAAGAGAGTTGTGGCAAA
GAGCCATGTTATGGATTAGTAATGGAAAGTATCGTCACCAATAGGGGAGTGGTCAATAATGGTCAATAA
CCCACACCTATAGGCTAAGCTATACCATCACCTATAACATGAGGAAGCGGGGGTGTATAGACCCCAAG
CCAAAAACAGTATAGCATGCATAAGAAGCCAAGGGGGTGGGCCTATAGACTCTATAGGCGGTACTTAC
GTCACTCTTGGCACGGGGAATCCGCGTTCCAATGCACCGTTCCCGGCCGCGGAGGCTGGATCGGT
CCCGGTGTCTTCTATGGAGGTCAAAACAGCGTGGATGGCGTCTCCAGGCGATCTGACGGTTCACTA
AACGAGCTCTGCTTATATAGACCTCCCACCGTACACGCCTACCGCCCATTTGCGTCAATGGGGCGGA
GTTGTTACGACATTTTGGAAAGTCCCGTTGATTTTGGTGCCAAAACAAACTCCCATTGACGTCAATGG
GGTGGAGACTTGGAAATCCCCGTGAGTCAAACCGCTATCCACGCCCATTGATGTACTGCCAAAACCG
CATCACCATGGTAATAGCGATGACTAATACGTAGATGTACTGCCAAGTAGGAAAGTCCCATAAGGTCAT
GTACTGGGCATAATGCCAGGCGGGCCATTTACCGTCATTGACGTCAATAGGGGGCGTACTTGGCATA

TGATACACTTGATGTACTGCCAAGTGGGCAGTTTACCGTAAATACTCCACCCATTGACGTCAATGGAA
AGTCCCTATTGGCGTTACTATGGGAACATACGTCATTATTGACGTCAATGGGCGGGGGTCGTTGGGC
GGTCAGCCAGGCGGGCCATTTACCGTAAGTTATGTAACGCGGAACTCCATATATGGGCTATGAACTAA
TGACCCCGTAATTGATTACTATTAATAACTAGTCAATAATCAATGTCAACATGGCGGTAATGTTGGACAT
GAGCCAATATAAATGTACATATTATGATATGGCGGCCGCATGATCCTGACGACGGAGACCGCGGTGGT
TGACCAGACAAACCTACATCGCGAAGCAGCGCAAAACGCCTAACCCTAAGCAGATTCTTCATGCAAT
TGTCGGTCAAGCCTTGCCTTGTTGTAGCTTAAATTTTGCTCGCGCACTACTCAGCGACCTCCAACAC
ACAAGCAGGGAGCAGATACTGGCTTAACTATGCGGCATCAGAGCAGATTGTACTGAGAGTCGACCAT
AGGGGATCGGGAGATCTCCCGATCCGTCGACGTCAGGTGGCACTTTTCGGGGAAATGTGCGCGGA
ACCCCTATTTGTTTATTTTTCTAAATACATTCAAATATGTATCCGCTCATGAGACAATAACCCTGATAAAT
GCTTCAATAATATTGAAAAAGGAAGAGTATGAGTATTCAACATTTCCGTGTCGCCCTTATTCCCTTTTTT
GCGGCATTTTGCCTTCCTGTTTTTGCTCACCCAGAAACGCTGGTGAAAGTAAAAGATGCTGAAGATC
AGTTGGGTGCACGAGTGGGTTACATCGAACTGGATCTCAACAGCGGTAAGATCCTTGAGAGTTTTCG
CCCCGAAGAACGTTTTCCAATGATGAGCACTTTTAAAGTTCTGCTATGTGGCGCGGTATTATCCCGTA
TTGACGCCGGGCAAGAGCAACTCGGTCGCCGCATACACTATTCTCAGAATGACTTGGTTGAGTACTC
ACCAGTCACAGAAAAGCATCTTACGGATGGCATGACAGTAAGAGAATTATGCAGTGCTGCCATAACCA
TGAGTGATAACACTGCGGCCAACTTACTTCTGACAACGATCGGAGGACCGAAGGAGCTAACCGCTTT
TTTGCACAACATGGGGGATCATGTAACTCGCCTTGATCGTTGGGAACCGGAGCTGAATGAAGCCATA
CCAAACGACGAGCGTGACACCACGATGCCTGTAGCAATGGCAACAACGTTGCGCAAACTATTAACTG
GCGAACTACTTACTCTAGCTTCCCGGCAACAATTAATAGACTGGATGGAGGCGGATAAAGTTGCAGG
ACCACTTCTGCGCTCGGCCCTTCCGGCTGGCTGGTTTATTGCTGATAAATCTGGAGCCGGTGAGCG
TGGGTCTCGCGGTATCATTGCAGCACTGGGGCCAGATGGTAAGCCCTCCCGTATCGTAGTTATCTAC
AAGAGAAACTCAGTCTGGTTTCACTCAGGCATGAACCCAAGCTTATGCTTTGTAAAGTTCAGCTTTTA
TAGGTCAGGTCACAGTGTGACATCTGAGGCTGGTCTCTGTTTCCACCATAGTTAGACATGTCTGCAG
GAACAAACATTTGCAGATAGAAATTGGAACACCCAGGCGCTTGCAGATACCAAATAAAGAATTCCTTC
CTTGGTGAACCTTTGATAGTTGCCAGAATTATTCCTTAAGGTAGTTTATGTGAATGTTTGCCTTCCCAA
GACAAGAGGAGGGGGGCAGAGTGAACTCAGCACATGAGTAAA

The genome of the product cell strain was amplified by using the above breakpoint forward primer F1 (SEQ ID No. 4): AGACCAGCCTCAGATGTCACAC and the reverse primer R2 (SEQ ID No. 10): TACAAATGTGGTATGGCTGATTAGC of the product gene on the plasmid used for the product to obtain a fragment consistent with the theoretical size (3665 bp), as shown in FIG. 7 (the band corresponding to lane GBB003-1D1).

The amplified band was cut and recovered from the gel, and purified, and multiple pairs of primers were designed for DNA sequencing. After splicing the sequencing results, the sequence was as shown in SEQ ID No. 9, which was consistent with the theoretical sequence (sequence SEQ ID No. 9), thus confirming that the target gene was correctly integrated into the target site. In the above, the sequence fragment after integrating the target gene 3 includes the attL. After integrating the target gene 3 into the cell strain with high expression of the target gene, the marker gene EGFP was replaced by the fragment where the target gene 3 was located.

### Reference:

[1] Takeshi Omasa et al,. Cell engineering and cultivation of chinese hamster ovary (CHO) cells. Curr Pharm Biotechnol. 2010 Apr;11(3):233-40.

The technical features of the above-mentioned embodiments and examples may be combined in any appropriate manner. For the sake of brevity, not all possible combinations of the various technical features in the above-mentioned embodiments and examples have been described. However, as long as there is no contradiction in these combinations of technical features, they should be considered to be within the scope recorded in this specification.

The above-mentioned examples only express several implementations of the present application, which is convenient for understanding the technical solutions of the present application in detail. However, it should not be construed as limiting the scope of patent protection of the present application. It should be noted that for a person ordinarily skilled in the art, several variations and improvements may be made without departing from the concept of the present application, which all belong to the protection scope of the present application. Additionally, it should be understood that after reading the above teachings of the present application, those skilled in the art may make various changes or modifications to the present application, and the equivalent forms obtained also fall within the protection scope of the present application. It should also be understood that the technical solutions obtained by those skilled in the art through logical analysis, reasoning or limited experiments are all within the protection scope of the claims appended to the present application. Hence, the scope of protection of the present application should be based on the contents of the appended claims, and the description and drawings may be used to interpret the contents of the claims.

## Claims

1. A nucleic acid, **characterized in that** the nucleic acid comprises a nucleic acid fragment as shown in SEQ ID No. 1, and the nucleic acid fragment is used to integrate an exogenous nucleic acid fragment.

2. The nucleic acid according to claim 1, wherein an integration site of the exogenous nucleic acid fragment corresponds to any site within a base interval from positions 11 to 430 of the nucleic acid fragment;
optionally, the integration site of the exogenous nucleic acid fragment corresponds to any site within the base interval from positions 21 to 414 of the nucleic acid fragment;
optionally, the integration site of the exogenous nucleic acid fragment corresponds to any site within the base interval from positions 38 to 402 of the nucleic acid fragment;
optionally, the integration site of the exogenous nucleic acid fragment corresponds to any site within the base interval from positions 53 to 389 of the nucleic acid fragment.;
optionally, the integration site of the exogenous nucleic acid fragment corresponds to any site within the base interval from positions 73 to 373 of the nucleic acid fragment;
optionally, the integration site of the exogenous nucleic acid fragment corresponds to any site within the base interval from positions 91 to 360 of the nucleic acid fragment;
optionally, the integration site of the exogenous nucleic acid fragment corresponds to any site within the base interval from positions 108 to 342 of the nucleic acid fragment;
optionally, the integration site of the exogenous nucleic acid fragment corresponds to any site within the base interval from positions 126 to 326 of the nucleic acid fragment;
optionally, the integration site of the exogenous nucleic acid fragment corresponds to any site within the base interval from positions 143 to 310 of the nucleic acid fragment;
optionally, the integration site of the exogenous nucleic acid fragment corresponds to any site within the base interval from positions 160 to 295 of the nucleic acid fragment;
optionally, the integration site of the exogenous nucleic acid fragment corresponds to any site within the base interval from positions 178 to 274 of the nucleic acid fragment;
optionally, the integration site of the exogenous nucleic acid fragment corresponds to any site within the base interval from positions 194 to 263 of the nucleic acid fragment;
optionally, the integration site of the exogenous nucleic acid fragment corresponds to any site within the base interval from positions 209 to 253 of the nucleic acid fragment;
optionally, the integration site of the exogenous nucleic acid fragment corresponds to any site within the base interval from positions 221 to 242 of the nucleic acid fragment; and
optionally, the integration site of the exogenous nucleic acid fragment corresponds to any site within the base interval from positions 231 to 240 of the nucleic acid fragment.

3. The nucleic acid according to claim 2, wherein the exogenous nucleic acid fragment comprises:
a first recombination recognition sequence and a second recombination recognition sequence, a selection marker gene and/or a target gene located between the first recombination recognition sequence and the second recombination recognition sequence, and a promoter for regulating an expression of the selection marker gene and/or the target gene;
optionally, the exogenous nucleic acid fragment comprises the selection marker gene located between the first recombination recognition sequence and the second recombination recognition sequence, and the promoter for regulating the expression of the selection marker gene;
optionally, the exogenous nucleic acid fragment comprises the target gene located between the first recombination recognition sequence and the second recombination recognition sequence, and the promoter for regulating the expression of the target gene; and
optionally, the exogenous nucleic acid fragment comprises the selection marker gene and the target gene located between the first recombination recognition sequence and the second recombination recognition sequence, and the promoter for regulating the expression of the selection marker gene and the target gene.

4. The nucleic acid according to claim 3, wherein the first recombination recognition sequence and the second recombination recognition sequence are generated by a recombinase; the recombinase is a Bxb1 integrase, a ΦC31 integrase, a Cre recombinase or a FLP recombinase;
or/and
the first recombination recognition sequence and the second recombination recognition sequence are each independently selected from one or more of following sequences: a LoxP sequence, a LoxPL3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxb1 attP sequence, a Bxb1 attB sequence, an attP sequence, an attB, an attL and an attR sequence.

5. The nucleic acid according to claim 3, wherein the selection marker gene is one or more selected from a neomycin resistance gene, a thymidine kinase gene, a hygromycin phosphotransferase gene, a dihydrofolate reductase gene, a thymidine kinase gene, a glutamine synthetase gene, an asparagine synthetase gene, a tryptophan synthetase gene, a histamine alcohol dehydrogenase gene, an aminoglycoside phosphotransferase gene, a tryptophan synthetase gene and a fluorescent protein gene; and
optionally, the selection marker gene is the fluorescent protein gene.

6. The nucleic acid according to claim 3, wherein the promoter is a CMV promoter, a SV40 promoter, an RSV promoter, a β-globin promoter, a UBC promoter, an EF1a promoter, a ubiquitin promoter, a β-actin promoter, a PGK1 promoter, a Rosa26 promoter, a HSP70 promoter, a GAPDH promoter, an Eif4A1 promoter, an Egr1 promoter, a FerH promoter, a SM22α promoter or an Endothelin-1 promoter.

7. The nucleic acid according to claim 3, wherein the target gene encodes one or more of an antibody, a recombinant protein, a polypeptide, an enzyme, a hormone, a growth factor and a receptor.

8. The nucleic acid according to any one of claims 1 to 7, wherein a nucleotide sequence of the nucleic acid comprises a nucleotide sequence as shown in SEQ ID No. 3, SEQ ID No. 7 or SEQ ID No. 9.

9. A recombinant vector, wherein the recombinant vector comprises the nucleic acid according to claim 1 or 2, and
the nucleic acid further comprises a first recombination recognition sequence and a second recombination recognition sequence; and the exogenous nucleic acid fragment comprises a target gene and/or a selection marker gene.

10. The recombinant vector according to claim 9, wherein the exogenous nucleic acid fragment is the target gene; the first recombination recognition sequence is a 5' homology arm homologous to a sequence fragment present in the nucleic acid fragment as shown in SEQ ID No. 1, and the second recombination recognition sequence is a 3' homology arm homologous to a sequence fragment present in the nucleic acid fragment as shown in SEQ ID No. 1; or,
the exogenous nucleic acid fragment is the selection marker gene; the first recombination recognition sequence is the 5' homology arm homologous to the sequence fragment present in the nucleic acid fragment as shown in SEQ ID No. 1; and the second recombination recognition sequence is the 3' homology arm homologous to the sequence fragment present in the nucleic acid fragment as shown in SEQ ID No. 1.

11. The recombinant vector according to claim 10, wherein the recombinant vector is a lentiviral vector, an adenoviral vector, an adeno-associated viral vector, a herpesvirus vector, a poxvirus vector, a baculovirus vector, a papillomavirus vector, a papovavirus vector, an integrating phage vector, a non-viral vector, a transposon and/or a transposase, an integrase substrate or a plasmid.

12. A targeted integrated cell, wherein the targeted integrated cell comprises the nucleic acid according to any one of claims 1 to 8.

13. The targeted integrated cell according to claim 12, wherein the targeted integrated cell is a eukaryotic cell;
optionally, the eukaryotic cell is a mammalian cell; and
optionally, the mammalian cell comprises a Chinese hamster ovary CHO cell and a human embryonic kidney HEK293 cell.

14. A preparation method for the targeted integrated cell according to claim 12 or 13, wherein the preparation method comprises following steps:
introducing the nucleic acid according to any one of claims 1 to 8 into a cell, or providing a cell comprising a nucleic acid fragment as shown in SEQ ID No. 1 and integrating the exogenous nucleic acid fragment into the nucleic acid fragment to prepare the targeted integrated cell.

15. A method for producing an expression product of a target gene, wherein the method comprises following steps: culturing the targeted integrated cell according to claim 12 or 13, and collecting the expression product of the target gene in the exogenous nucleic acid fragment.

16. Use of the nucleic acid according to any one of claims 1 to 8 in preparing a protein or a polypeptide, wherein the protein or the polypeptide is expressed by the exogenous nucleic acid fragment.
